# EUROPÄISCHE PATENTSCHRIFT

(11) **EP 3 620 801 B1**
(45) Veröffentlichungstag und Bekanntmachung des Hinweises auf die Patenterteilung: **03.03.2021**
(21) Anmeldenummer: 18193140.3
(22) Anmeldetag: 07.09.2018
(51) Int. Cl.: G01R 27/22, A61B 18/02, A61B 18/14, A61B 18/00, A61B 18/12, A61B 18/16

(54) **GERÄT ZUR SPEISUNG EINES MEDIZINISCHEN INSTRUMENTS UND VERFAHREN ZUR INSTRUMENTENÜBERWACHUNG**
DEVICE FOR FEEDING A MEDICAL INSTRUMENT AND METHOD FOR INSTRUMENT MONITORING
APPAREIL D'ALIMENTATION D'UN INSTRUMENT MÉDICAL ET PROCÉDÉ DE SURVEILLANCE DES INSTRUMENTS

(43) Veröffentlichungstag der Anmeldung: 11.03.2020
(73) Patentinhaber: Erbe Elektromedizin GmbH, 72072 Tübingen (DE)
(72) Erfinder: Fritz, Martin Georg, 72070 Tübingen (DE)
(74) Vertreter: Rüger Abel Patentanwälte PartGmbB

(56) Entgegenhaltungen:
- WO-A1-2012/017731
- DE-A1- 19 961 744
- US-A1- 2015 141 847
- LAUGIER P ET AL: "A NEW ECHOGRAPHIC CRYOPROBE FOR IN VIVO ULTRASONIC MONITORING OF SKIN CRYOSURGERY", 1998 IEEE ULTRASONICS SYMPOSIUM PROCEEDINGS. SENDAI, MIYAGI, JP, OCT. 5 - 8, 1998; [IEEE ULTRASONICS SYMPOSIUM PROCEEDINGS], NEW YORK, NY : IEEE, US, 5. Oktober 1998 (1998-10-05), Seiten 1337-1340, XP000871838, ISBN: 978-0-7803-4096-1

## Beschreibung

Die Erfindung betrifft ein Gerät zur Speisung eines über eine Leitung an das Gerät angeschlossenen medizinischen Instruments mit Signal- oder Betriebsleistung und/oder mit einem Betriebsmedium sowie ein Verfahren zur Überwachung eines über die Leitung von dem Gerät gespeisten medizinischen Instruments.

Aus der EP 2 520 241 B1 ist eine Einrichtung bestehend aus einem medizinischen Instrument zur Behandlung eines Patienten und einem Gerät zur Speisung des Instruments mit Behandlungsstrom bekannt. Das Gerät misst während der Behandlung den durch Gewebe des Patienten fließenden elektrischen Strom sowie die an dem Instrument anliegende elektrische Spannung und nutzt die gewonnenen Strom- und Spannungswerte zur Steuerung des Geräts.

Mit einem etwas anderen Steueralgorithmus nutzt die Einrichtung gemäß EP 2 520 240 B1 ebenfalls den von einem Instrument durch biologisches Gewebe geleiteten Strom sowie die an dem Gewebe bzw. dem Instrument anliegende Spannung zur Steuerung des Geräts.

Aus der EP 1 064 532 B1 ist ein Verfahren zur Messung der Blutgerinnungszeit einer Blutprobe eines Patienten bekannt, wobei das Blut in einer Testkammer über elektrische Kontakte mit Testsignalen eines Spannungsgenerators beaufschlagt wird, so dass ein Strom durch das Blut fließt, der sich entsprechend der Blutgerinnung verändert.

Die genannten Einrichtungen und Verfahren sind jeweils speziell auf bestimmte Messungen an biologischen Geweben eingerichtet. Weitere Einrichtungen sind aus WO2012/01773, US2015/141847, DE1996744 und dem Artikel 'A NEW ECHOGRAPHIC CRYOPROBE FOR IN VIVO ULTRASONIC MONITORING OF SKIN CRYOSURGERY', Laugier et al, 1998 IEEE ULTRASONICS SYMPOSIUM PROCEEDINGS. SENDAI, MIYAGI, JP, OCT. 5 - 8, 1998, bekannt.

Es ist Aufgabe der Erfindung, ein Konzept zur elektrischen Überwachung von medizinischen Instrumenten sowie deren zugehörigen Komponenten sowie des Betriebs derselben anzugeben.

Das erfindungsgemäße Konzept ist in einem Gerät zur Speisung eines über eine Leitung an das Gerät angeschlossenen medizinischen Instruments mit Signalen oder Betriebsleistung und/oder einem Betriebsmedium verwirklicht, bei dem ein Testimpulssender vorgesehen ist, der dazu eingerichtet ist, an die das Gerät mit dem Instrument verbindende Leitung Testsignale abzugeben. Außerdem weist das Gerät einen Echoempfänger auf, der dazu eingerichtet ist, das über die Leitung in Reaktion auf das Testsignal zurückkommende Echosignal zu empfangen. Das Echosignal wird einer Analyseeinrichtung zugeführt, die dazu eingerichtet ist, anhand des Echosignals eine Veränderung eines physikalischen Zustands in oder an dem Instrument und/oder in oder an der Leitung zu erfassen. Das Testsignal ist vorzugsweise gleichspannungsfrei und kann, je nach Anwendungsfall, eine Spitzenspannung von wenigen Volt bis zu mehreren 1000 V aufweisen.

Die Leitung ist eine Fluidleitung, insbesondere eine Metallkapillare oder ein Fluidschlauch, der ein oder mehrere elektrische Leiter enthält oder selbst ein elektrischer Leiter ist. Das von dem Testsignalsender an die Leitung abgegebene Testsignal läuft dann auf oder in der Leitung zu dem Instrument und wird von diesem ganz oder teilweise absorbiert und/oder ganz oder teilweise reflektiert. Das reflektierte Echosignal trifft seiner Laufzeit entsprechend verzögert an dem Echosignalempfänger ein und wird von diesem an die Analyseeinrichtung weitergegeben. Die Analyseeinrichtung kann nun anhand der Laufzeit sowie der Signalverformung (Verzerrung) oder anderer Eigenschaften des Echosignals, wie z.B. Phasenlage, Amplitude, Hüllkurve und Ähnliches, auf den Zustand der Leitung (z.B. deren Länge oder Integrität, auf den physikalischen Zustand in oder an dem Instrument, beispielsweise die Betätigung eines Aktivierungsschalters, den Kontakt zu biologischem Gewebe oder den Zustand desselben schließen, woraufhin das Gerät entsprechend gesteuert werden kann. Beispielsweise kann das Instrument ein kryochirurgisches Instrument sein, bei dem das Testsignal bis zur Kryospitze läuft und an dieser zu dem Gerät zurück reflektiert wird. Bei Vereisungsbeginn an der Kryospitze erfahren charakteristische Parameter des Echosignals eine Veränderung, beispielsweise hinsichtlich seiner Phasenlage, so dass der Vereisungsbeginn erkannt und die Vereisungszeit vom Vereisungsbeginn an gerechnet werden kann. Eisbildung in oder an der Leitung kann ebenfalls Einfluss auf das Echosignal haben. Z.B. können Mehrfachechos entstehen und deren Vorhandensein als Kriterium zur Erzeugung eines entsprechenden das Event indizierenden Signals genutzt werden. Andere Anwendungen sind möglich. Z.B. ist mit dem erfindungsgemäßen Konzept bei Kryoinstrumenten eine Autostarterkennung möglich, d.h. ein selbständiger Behandlungsstart, abhängig von definierten Zuständen am Instrument.

Das Echosignal kann von physikalischen Zuständen an dem Instrument abhängig sein, wie beispielsweise dem Zustand und/oder der Temperatur einer Elektrode, der elektrischen Kapazität der Elektrode zum Patienten oder zur Gegenelektrode, einem elektrischen Widerstand zwischen der Elektrode und der Gegenelektrode, der elektrischen Induktivität des Signalwegs, einer elektrischen Impedanz, eienr Leitfähigkeit eines an der Elektrode vorhandenen Fluids, insbesondere Gases, der Berührung der Leitung durch einen anderen Körper oder Gegenstand und dergleichen mehr.

Die physikalischen Zustände können Eigenschaften des Echosignals beeinflussen. Solche Eigenschaften können z.B. die Laufzeit, die Anzahl von Echosignalen, die Echosignalamplitude, die Verzerrung des Testsignals (d.h. seine Kurvenform) oder die Phasenlage des Echosignals in Bezug auf das Testsignal sein, das Vorhandensein oder Verschwinden des Echosignals sein.

Das Gerät kann eine Steuereinrichtung aufweisen, die auf wenigstens eines oder auf mehrere dieser erfassten physikalischen Zustände mit einer Aktion reagiert, wie beispielsweise dem Ein- und Ausschalten des Generators, der Erhöhung oder Verminderung der Leistung des Generators, der Spitzenspannung, des Behandlungsstroms, der Impulsdauer des Behandlungsstroms oder den Pausen zwischen einzelnen Behandlungsstromimpulsen, oder dergleichen mehr. Zu den in Reaktion auf eine charakteristische Veränderung des Echosignals ausgelösten Aktionen kann auch das Aktivierung oder Deaktivieren des Instruments und/oder des speisenden Geräts gehören. Die Aktivierung oder Deaktivierung des speisenden Geräts bedeutet die Freigabe oder Sperrung der Betriebsleistung oder des Betriebsmediums.

Der Testsignalsender ist vorzugsweise dazu eingerichtet, gleichspannungsfreie und/oder gleichstromfreie Testsignale zu generieren. Solche Testsignale sind beispielsweise modulierte HF-Signale, z.B. mit einer glockenförmigen Hüllkurve modulierte HF-Signale, wobei die Dauer eines solchen Testsignals vorzugsweise wenige Nanosekunden beträgt. Ein solches Signal mit glockenförmiger Hüllkurve kann ein Hochfrequenzsignalburst sein wird als einzelner Testsignalimpuls angesehen. Es ist jedoch auch möglich, anderweitig modulierte HF-Signale oder Impulssignale als Testsignale zu nutzen, beispielsweise insbesondere gleichspannungsfreie Impulssignale nach Art von Dirac-Impulsen, Dreieckimpulsen, Sägezahnimpulsen, Rechteckimpulsen, Sinc-Impulsen oder an solche Impulse angenäherte Impulse. Unter Sinc-Impulsen werden Impulse der Form (sin x)/x oder Pulse verstanden, die aus einem oder mehreren solcher Pulse abgeleitet sind.

Durch die Einleitung von Testimpulsen in die Leitung und die Ausleitung von Echosignalen vorzugsweise durch induktive und oder kapazitive Kopplung eines Messtromkreises an die Leitung, z.B. über Richtkoppler, sind die Steuerungselektronik und der Patientenstromkreis galvanisch getrennt.

Das erfindungsgemäße Verfahren dient der Überwachung eines über eine Leitung von dem Gerät gespeisten medizinischen Instruments, wobei auf die das Gerät mit dem Instrument verbindende Leitung mindestens ein Testsignal gegeben, das über die gleiche Leitung zurückkommende Echosignal empfangen und dieses dann einer Analyse unterzogen wird, wobei aus der Analyse eine Veränderung eines physikalischen Zustands in oder an dem Instrument sowie gegebenenfalls auch in oder an der das Instrument speisenden Leitung erfasst wird. Durch die Auswertung der Eigenschaften des zeitversetzt zur Aussendung des Testsignals eintreffenden Echosignals ist es möglich, sowohl Funktion und Zustand der Leitung wie auch des Instruments und darüber hinaus des von dem Instrument beeinflussten biologischen Gewebes zu überwachen.

Weitere Einzelheiten vorteilhafter Ausführungsformen sind Gegenstand der Zeichnung, der Beschreibung oder von Ansprüchen. In der Zeichnung sind Ausführungsbeispiele der Erfindung veranschaulich. Es zeigen:
Figur 1 eine Einrichtung zur kryochirurgischen Behandlung eines Patienten mit einem kryochirurgischen Instrument und einem Gerät zur Speisung desselben, in schematisierter Darstellung,
Figur 2 das kryochirurgische Instrument und biologisches, von diesem beeinflusstes Gewebe, in schematisierter Darstellung,
Figur 3 einen Richtkoppler zum Ausleiten eines Echosignals aus der das Instrument speisenden Leitung, in schematisierter Darstellung,
Figur 4 - 6 verschiedene Testsignale und deren Echos, jeweils als schematisches Diagramm,
Figur 7 eine Einrichtung zur elektrochirurgischen Behandlung von biologischem Gewebe mit einem bipolaren Instrument und mit einem dieses speisenden Gerät, in schematisierter Darstellung, und
Figur 8 einen Richtkoppler zur Ausleitung des Echosignals aus einer das Instrument speisenden Leitung.

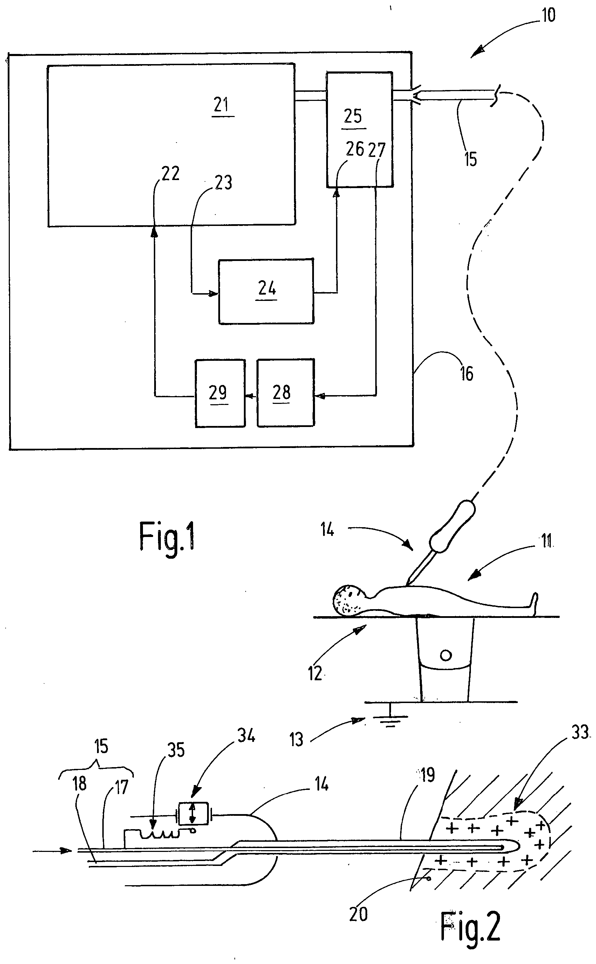

In Figur 1 ist eine kryochirurgische Einrichtung 10 dargestellt, die zur Einwirkung auf einen Patienten 11 dient. Dieser ist in Figur 1 rein exemplarisch auf einer Liege 12 liegend angeordnet, über die der Patient 11 mindestens kapazitiv mit Erdpotential 13 verbunden ist.

Zur Behandlung des Patienten 11 dient eine Kryosonde 14, die über eine Leitung 15 mit einem speisenden Gerät 16 verbunden ist. Die Leitung 15 ist typischerweise eine Fluidleitung, beispielsweise ein Kapillarrohr, ein Schlauch oder dergleichen. Über die Leitung 15 wird ein Behandlungsfluid von dem Gerät 16 zu dem Instrument 14 geleitet. Die Leitung 15 umfasst, wie Figur 2 erkennen lässt, sowohl eine Zufuhrleitung 17 als auch eine Rückführungsleitung 18. Erfindungsgemäß ist wenigstens eine der beiden Leitungen 17, 18 elektrisch leitend ausgebildet oder mit einem elektrischen Leiter versehen, so dass die Kryospitze 19, die zum direkten Kontakt mit biologischem Gewebe 20 dient, von einem elektrischen Testsignal erreichbar ist, das von dem Gerät 16 ausgehend über die Leitung 15 zu dem Instrument 14, insbesondere der Kryospitze 19, und von dieser über die Leitung 15 zurück zu dem Gerät 16 läuft. Das Testsignal ist vorzugsweise kürzer als seine Laufzeit, so dass das Testsignal und Echosignal am Leitungsanfang zeitversetzt vorhanden sind. Die Kryosonde 19 kann mit einer in der Leitung 15 enthaltenen elektrischen Leitung galvanisch oder auch lediglich kapazitiv koppeln.

Das Gerät 10 enthält eine Speiseeinrichtung 21, über die das Instrument 14 mit Betriebsmedium und/oder Betriebsleistung versorgt wird. In dem Ausführungsbeispiel nach Figur 1 handelt es sich bei dem Betriebsmedium um ein Fluid, beispielsweise Kohlendioxid oder Lachgas (N₂O), Stickstoff, z.B. als Gas, als Flüssigkeit, vorzugsweise nahe der Siedelinie, oder als Zwei-Phasen-Gemisch. Die Speiseeinrichtung 21 enthält einen entsprechenden Fluid-Vorrat oder ist mit einem solchen verbunden. Zur manuellen Steuerung, insbesondere zur Auslösung einer Behandlung, d.h. zur Signalisierung des Behandlungsbeginns, kann an dem Gerät 16 ein Steuereingang vorgesehen sein, der nicht weiter veranschaulicht ist. Die Speiseeinrichtung kann alternativ oder zusätzlich einen Steuereingang 22 aufweisen, über die sie ein Ein- oder Ausschaltsignal oder ein sonstiges Steuersignal empfangen kann. Außerdem kann die Speiseeinrichtung 21 einen Ausgang 23 aufweisen, über den sie einen Abfragebefehl an nachgeordnete Einrichtungen senden kann. Der Abfragebefehl kann dazu dienen, einen Messzyklus auszulösen, der zur Bestimmung eines physikalischen Zustands in oder an der Leitung 15 sowie in oder an dem Instrument 14 dient. An den Ausgang 23 ist bei dem Ausführungsbeispiel nach Figur 1 ein Testsignalsender 24 angeschlossen, der über eine Koppeleinrichtung 25 ein Testsignal an die Leitung 15 abgibt. Dieses Testsignal weist bei dieser und bei allen anderen nachfolgend beschriebenen Ausführungsformen eine Signaldauer auf, die vorzugsweise höchstens so groß ist, wie die Laufzeit des Testsignals auf der Leitung 15 zu dem Instrument 14 und als Echosignal zurück zu der Koppeleinrichtung 25.

Außer einem Signaleingang 26 weist die Koppeleinrichtung 25 einen Signalausgang 27 auf, der das auf der Leitung 15 ausgeleitete Echosignal bereitstellt und an einen Echosignalempfänger 28 übergibt. Dieser ist Teil einer Analyseeinrichtung 29 oder mit einer solchen verbunden, die das Echosignal untersucht und dem Untersuchungsergebnis entsprechend ein Steuersignal an den Steuereingang 22 abgibt.

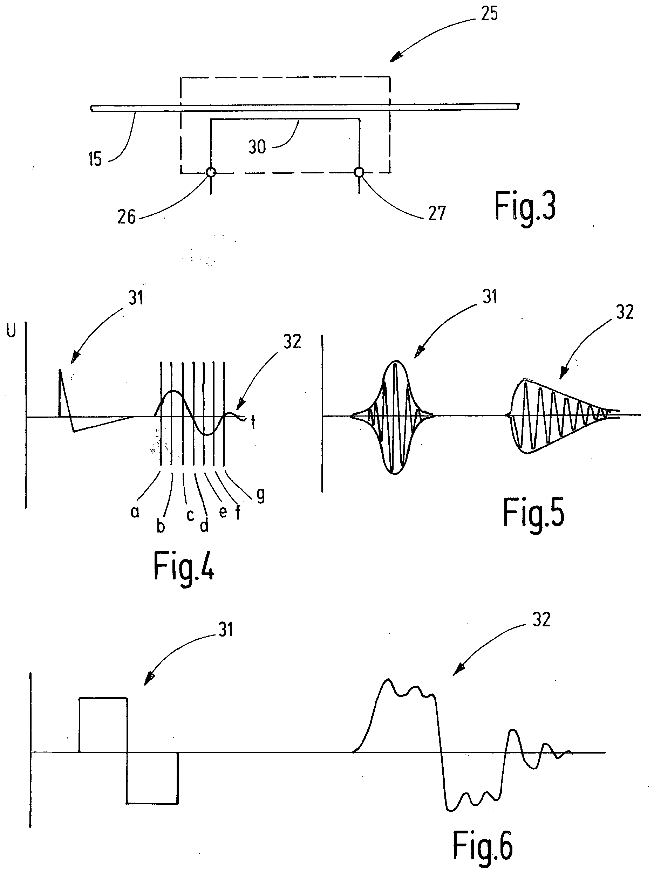

Figur 3 veranschaulicht die Koppeleinrichtung 25 schematisch und exemplarisch. Es handelt sich bei der Koppeleinrichtung 25 um einen Richtkoppler zum Ein- und Auskoppeln von elektrischen Signalen in die elektrisch leitende und als Fluidleitung ausgebildete Leitung 15. Dazu ist auf einem geeigneten Träger ein Leiterabschnitt 30 angeordnet, der auf ganzer Länge oder wenigstens an seinen Enden kapazitiv mit der Leitung 15 koppelt, während er eine von Null verschiedene Eigeninduktivität aufweist. An seinen beiden Enden kann die auf der Leitung 15 hin- und rücklaufende Welle eines elektrischen Signals, beispielsweise eines Testsignals 31, jeweils gesondert abgenommen werden. Zum Beispiel kann an dem Signaleingang 26 ein von dem Testsignalsender 24 generiertes Testsignal 31 eingespeist werden, wie es in Figur 4 schematisch veranschaulicht ist. Vorzugsweise ist die zeitliche Länge dieses Signals geringer als die Zeit, die das Testsignal 31 benötigt, um über die Leitung 15 zu dem Instrument 14 und als Echo 32 zu der Koppeleinrichtung 25 zurück zu laufen. Typischerweise ist das Testsignal 31 einige Nanosekunden lang.

Als Beispieltestsignal ist in Figur 4 ein nadelförmiger positiver Spannungsimpuls gefolgt von einem negativen Dreieckimpuls veranschaulicht. Die von dem positiven und dem negativen Teil des Testsignals umgrenzten Flächenbereiche sind vorzugsweise gleich groß, so dass das Testsignal 31 insgesamt gleichspannungsfrei ist.

Das Echosignal 32 weist eine veränderte Form auf, die in Figur 4 symbolisch veranschaulicht ist. Zum Beispiel können hochfrequente Anteile fehlen, während Überschwingungen auftreten, wobei das Echosignal auch zeitlich gedehnt oder gestaucht sowie in seiner Amplitude verändert, insbesondere verringert sein kann.

Vorzugsweise ist der Echosignalempfänger 28 darauf eingerichtet, von aufeinanderfolgenden Testsignalen 31 herrührende Echosignale 32 wiederholt, dabei aber zeitversetzt abzutasten. Diese Abtastzeitpunkte sind in Figur 4 durch senkrechte Linien a, b, c, d, e, f, g symbolisiert. Aus den zu den verschiedenen Zeitpunkten a bis g gewonnenen Abtastwerten rekonstruiert der Echosignalempfänger 28 das Echosignal. Die Anzahl der Abtastungen ist zweckentsprechend festgelegt. Insoweit ist Figur 4 lediglich beispielhaft.

Die insoweit beschriebene Einrichtung 10 arbeitet wie folgt:
Nach grundsätzlicher Aktivierung des Geräts 16 und Platzierung des Instruments 14 am oder im Gewebe 20 des Patienten 11, z.B. ähnlich Figur 2, wird die Speiseeinrichtung 21 freigegeben, so dass diese über die Leitung 15 Fluid zu der Kryospitze 19 liefert. Die Kryospitze 19 ist zunächst noch von feuchtem, lebendem Gewebe umgeben. Zuvor oder zugleich wird der Testsignalsender 24 aktiviert, der nun Testsignale 31, z.B. gemäß Figur 4, an die Koppeleinrichtung 25 und über diese an die Leitung 15 abgibt. Jedes Testsignal 31 läuft nun die Leitung 15 entlang zu der Spitze 19, wo es auf das zumindest kapazitiv geerdete Gewebe 20 trifft. Das elektrisch leitfähige Gewebe 20 schließt somit die aus der Leitung 15 und der Kryospitze 19 bestehende elektrische Leitung kapazitiv und resistiv ab. Entsprechend wird das Testsignal 31 abgeschwächt und, je nach dem an der Kryospitze vorhandenen elektrischen Widerstand, mit gleicher oder invertierter Phase reflektiert. Zugleich wird es in Folge des Kapazitätsbelags und des Induktivitätsbelags sowie des Einflusses des Gewebes 20 verzerrt, weswegen das Echosignal 32 eine andere Kurvenform aufweist als das Testsignal 31. Nach mehreren beispielsweise in wenigen Mikrosekunden aufeinanderfolgenden Messzyklen, die jeweils aus der Aussendung eines Testsignals 21 durch den Testsignalsender 24 und dem Empfang des Echosignals durch den Echosignalempfänger 28 bestehen, ist die Form des Echosignals 32 erfasst und kann von der Analyseeinrichtung 29 ausgewertet werden. Mit fortwährender Kühlung der Kryospitze 19 kann sich in dem Gewebe 20 ein gefrorener Bereich 33 ausbilden, der die physikalischen Eigenschaften in unmittelbarer Nachbarschaft der Sondenspitze 19 charakteristisch ändert. Z.B. nimmt die Stromleitfähigkeit ab. Damit ändert sich auch die Form des Echosignals 32 signifikant. Beispielsweise kann beginnende Eisbildung an der Kryospitze 19 dazu führen, dass das Ende der als Wellenleiter betrachteten Kryospitze 19 elektrisch als "offen" wirkt, während es vor der Ausbildung elektrisch als "kurzgeschlossen" anzusehen war. Entsprechend wechselt das Echosignal 32 bei beginnender Eisbildung seine Phase. Dieser Phasenwechsel kann von der Analyseeinrichtung 29 erfasst und ein entsprechendes Signal an den Steuereingang 22 gegeben werden. Das Signal kann zur Steuerung des Betriebs der Speiseeinrichtung 21 dienen. Sofern die Speiseeinrichtung 21 nun eine bestimmte definierte Zeit für die Eisbildung an der Kryospitze 19 vorsieht, kann die vorgegebene Gefrierzeit nun mit dem Eintreffen des Signals an dem Steuereingang 22 gestartet werden.

Das vorstehend beschriebene Ausführungsbeispiel dient der prinzipiellen Veranschaulichung. Die Analyseeinrichtung 29 kann jedoch auch dazu eingerichtet sein, wesentlich subtilere Analysen durchzuführen. Beispielsweise lassen sich anhand der Form des Echosignals 32 ein oder mehrere weitere oder andere physikalische Zustände erfassen, beispielsweise die Temperatur der Kryosonde 19 und/oder des Gewebes 20, die Größe des gefrorenen Gewebes 33, der Typ einer an dem Instrument 14 befestigten Kryospitze 19, die Länge der Leitung 15 und dergleichen mehr.

Es sind weitere Anwendungen des erfindungsgemäßen Konzepts möglich. Beispielsweise kann die Kryospitze 19 elektrisch von der Fluidzufuhrleitung 17 und somit auch von der Leitung 15 isoliert sein. Gleiches kann für die Fluidrückführungsleitung 18 gelten. In diesem Fall kann das erfindungsgemäße Prinzip dazu verwendet werden, eine an dem Instrument 14 manuell herbeigeführte Veränderung zu erfassen. Beispielsweise kann dazu ein elektrisch leitendes Bedienelement 34 vorgesehen sein, das mit der Fluidzufuhrleitung 17 oder mit der Fluidrückführungsleitung 18 (oder mit beiden) in und außer Eingriff überführbar ist, so dass es den Kapazitätsbelag der Leitung 15 oder der Kryospitze lokal beeinflusst. Außerdem kann das Bedienelement 34 elektrisch leiten und so galvanisch mit der Bedienperson verbunden sein, sobald diese es berührt. Es kann mit der Leitung 15 verbunden oder von dieser getrennt werden, je nachdem, wie eine Bedienperson das Bedienelement 34 betätigt. Ist das Bedienelement 34 von der Leitung 15 elektrisch getrennt, hat das Echosignal 32 eine andere Form als in dem Fall, in dem es mit der Leitung 15 verbunden ist. Die entsprechende Signalveränderung kann von der Analyseeinrichtung 29 dazu genutzt werden, die Speiseeinrichtung 21 ein- und auszuschalten.

Die zuletzt beschriebene Ausführungsform mit Bedienelement 34 lässt sich auch mit der zuvor beschriebenen Ausführungsform kombinieren, bei der die Kryospitze 19 elektrisch mit der Leitung 15 verbunden ist. Z.B. können bei Betätigung des Bedienelements auftretende Mehrfachechos als Indikator für die Betätigung des Bedienelements 34 genutzt werden.

Es ist auch möglich, zwischen dem Bedienelement 34 und der Leitung 15 ein weiteres Element anzuordnen, wie beispielsweise eine Induktivität 35 oder, wie in Figur 2 angedeutet, einen Parallelschwingkreis, einen Reihenschwingkreis oder dergleichen. Die Elemente oder Schwingkreise können das Testsignal jeweils auf charakteristische Weise beeinflussen und entsprechend charakteristische Echosignale verursachen.

Werden an dem Instrument 14 mehrere solcher Schwingkreise oder sonstige elektrische Elemente und mehrere Bedienelement dazu angeordnet, können über die verschiedenen damit erreichbaren Veränderungen des Echosignals 32 verschiedene Befehle an die Speiseeinrichtung übermittelt werden.

Für alle vor- und nachfolgend beschriebenen Ausführungsformen gilt, dass diese mit dem Testsignal 31 nach Figur 4 sowie alternativ auch mit anderen Testsignalen betrieben werden können, wie sie rein beispielhaft in den Figuren 5 und 6 veranschaulicht sind. Als vorteilhaftes Testsignal wird ein mit einer Gaußkurve amplitudenmoduliertes HF-Signal verstanden, wie es in Figur 5 veranschaulicht ist. Es ist gleichspannungsfrei.

Anstelle eine amplitudenmodulierten HF-Signals kann auch ein Sinc-Signal genutzt werden, das als Einzelpuls oder als Folge zweier oder mehrere Sinc-Pulse mit unterschiedlicher Polarität bereitgestellt wird.

Das Echosignal 32 nach Figur 4 weist typischerweise eine veränderte Hüllkurve auf, die für verschiedene physikalische Zustände an dem Instrument 14 charakteristisch ist. Die physikalischen Zustände können dabei sowohl das beeinflusste biologische Gewebe 20 als auch andere Zustände, wie beispielsweise die Berührung oder Betätigung eines Bedienelements 34 (oder weitere Bedienelemente) betreffen. Außer der Hüllkurve kann auch die Phasenlage der von der Hüllkurve modulierten HF-Schwingung ausgewertet werden. Alle diese Varianten sind mögliche Ausgestaltungen des Echosignalempfängers 28 und der Analyseeinrichtung 29.

Figur 6 veranschaulicht ein weiteres mögliches Testsignal, beispielsweise in Gestalt eines positiven und mit Pause oder unmittelbar darauf folgenden negativen Rechteckimpulses. Das zugehörige Echosignal 32 kann verringerte Flankensteilheit, Überschwinger, eine Phasendrehung und dergleichen weitere Veränderungen in Bezug auf das Testsignal 31 aufweisen. Jede Veränderung des Echosignals 32 im Vergleich zu dem Testsignal 31 kann als Charakteristikum für eine an der Leitung 15 und/oder dem Instrument 14 aufgetretene Änderung der physikalischen Bedingungen verstanden und von der Analyseeinrichtung 29 entsprechend ausgewertet werden.

Anstelle der Rechteckpulse nach Figur 6 können als Testsignal 31 auch dreieck- oder trapezförmige Impulse dienen. Weitere Signalformen sind möglich.

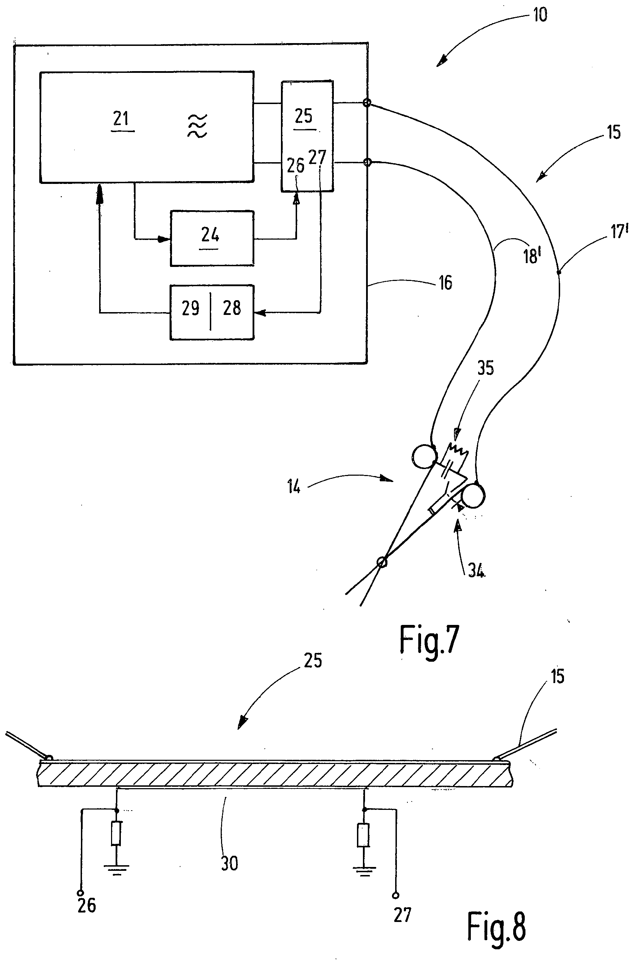

Ein nicht beanspruchtes Beispiel betrifft andere Einrichtungen 10, bei denen ein Gerät 16 ein monopolares oder bipolares Instrument 14 mit einem Medium oder auch mit Betriebsleistung, beispielsweise mit elektrischem Strom oder elektrischer Spannung versorgt. Zur Veranschaulichung ist in Figur 7 ein bipolares Instrument 14 dargestellt, dessen Speiseeinrichtung 21 ein HF-Generator ist. Das Instrument 14 ist symbolisch als Kauterisierungszange veranschaulicht, wobei jede Bauform eines bipolaren elektrischen Instruments 14 vorgesehen werden kann. Die Leitung 15 umfasst eine elektrische Zufuhrleitung 17' und eine elektrische Rückführungsleitung 18', die zusammen einen Wellenleiter bilden. Zum Beispiel sind die Leitungen 17', 18' an die beiden Branchen der Kauterisierungszange angeschlossen. Zusätzlich kann ein Bedienelement 34 beispielsweise in Gestalt eines elektrischen Schalters vorgesehen sein, mit dem die Leitungen 17', 18' über ein Element untereinander verbindbar sind, das die Wellenleitereigenschaften der Leitung 15 lokal ändern kann. Das Element kann z.B. eine Induktivität 35 oder wie dargestellt ein Schwingkreis sein. Der Schwingkreis kann ein Parallelschwingkreis, ein Reihenschwingkreis oder ein Bauelement sein, das sowohl kapazitive als auch induktive Eigenschaften in sich vereint. Alternativ kann ein resistives Element vorgesehen sein, beispielsweise ein Widerstand, der dem Wellenwiderstand der Leitung 15 entspricht. In diesem Fall bewirkt das Schließen des Schalters 34 eine Absorption des Testsignals 31, so dass das Echosignal 32 entfällt.

Die Koppeleinrichtung 25 kann gemäß Figur 8 ein Richtkoppler sein, der beispielsweise als Koaxialanordnung oder auch als Leiterstreifen auf einer Leiterplatte ausgebildet ist. Zum Beispiel kann die den Behandlungsstrom führende Leitung 15 auf einer Seite einer Leiterplatte angeordnet sein, während der Leiterabschnitt 30 auf der Gegenseite derselben angeordnet ist. So wird auf einfache Weise eine hohe elektrische Isolationsfestigkeit und somit eine sichere Trennung zwischen dem von dem elektrischen Generator der Speiseeinrichtung 21 zu dem Instrument 14 führenden Stromkreis und dem von dem Testsignalsender 24 zu dem Testsignalempfänger 28 führenden Testsignalkreis erreicht.

Wiederum lässt sich mit der Einrichtung 10 eine Vielzahl von Varianten realisieren. Beispielsweise kann die Analyseeinrichtung 29 durch geeignete Auswertung des Echosignals 32 Beginn oder Ende einer Kauterisierung oder auch eine erfolgreiche Gewebetrennung sowie eine Betätigung eines etwaigen vorhandenen Bedienelements 34 erfassen.

Das Gleiche gilt auch für monopolare Instrumente, bei denen lediglich die Zuführungsleitung 17' von dem Gerät 16 zu dem Instrument 14 führt, während die Rückführungsleitung 18' von einer am Patienten befestigten Neutralelektrode zu dem Gerät 16 führt. Auch hier läuft das Testsignal 31 über die Zuführleitung 17' von dem Gerät 16 zu dem Instrument 14 und das Echosignal 32 läuft auf der gleichen Zuführleitung 17' von dem Instrument 14 zu dem Generator 16 zurück. Wiederum ist die Veränderung des Echosignals 32 im Vergleich zu dem Testsignal 31 ein Indikator für die physikalischen Gegebenheiten auf der Zuführleitung 17' und an dem Instrument 14, so dass entsprechende Änderungen des Echosignals zur Auslösung von Aktionen, wie beispielsweise dem Ein- und Ausschalten der Speiseeinrichtung, die Erhöhung oder Minderung von deren Spannung, Leistung oder Strom und/oder die Änderung der Signalform der von der Speiseeinrichtung 21 abgegebenen Spannung genutzt werden können.

Bei allen Einrichtungen 10, bei denen die Speiseeinrichtung 21 zur Abgabe eines elektrischen Behandlungsstroms eingerichtet ist, erfolgt das Senden von Testsignalen 31 unter Empfang der Echosignale 32 vorzugsweise während kurzer Pausen, in denen die Speiseeinrichtung 21 kein Leistungssignal an die Leitung 15 abgibt. Dazu wird der Betrieb des Generators der Speiseeinrichtung 21 vorzugsweise repetierend kurz unterbrochen. Beispielsweise ist der Generator ein HF-Generator, der mit einer Grundfrequenz von mehreren 100 kHz (z.B. 350 oder 400 kHz) schwingt, wobei er mit einer Frequenz von mehreren 10 kHz (z.B. 46 kHz) pulsweitenmoduliert ist. Damit ist das von dem HF-Generator abgegebene HF-Signal zum Beispiel rechteckmoduliert, d.h. eine Folge aufeinanderfolgender HF-Schwingungspakete. Jedes HF-Schwingkungspaket besteht aus mindestens einer, ggfs. auch mehreren oder vielen HF-Schwingungen. Das Senden des Testsignals 21 und Empfangen des Echosignals 32 findet vorzugsweise in den Pausen zwischen zwei aufeinanderfolgenden HF-Schwingungspaketen statt.

Mit dem vorgestellten Konzept lassen sich nicht nur Eigenschaften des Instruments 14 und Eigenschaften des Gewebes 20, sondern auch Eigenschaften von eine Elektrode des Instruments umgebenden Fluiden, insbesondere Gasen oder Plasmen, bestimmen. Beispielsweise kann bei einem mit Funkenentladung arbeitenden Instrument in den Pausen zwischen zwei HF-Schwingungspaketen mittels Testimpulsen der Ionisierungszustand des an der Elektrode vorhandenen Gases ermittelt und für einen Betrieb der Speiseeinrichtung 21 herangezogen werden. Die Testimpulse können Spannungsamplituden von über 1000V haben. Beispielsweise kann an einem mono- oder bipolaren Koagulationsinstrument die Pause zwischen zwei aufeinanderfolgenden HF-Schwingungspaketen verkürzt werden, wenn in der Pause zwischen zwei aufeinanderfolgenden HF-Schwingungspaketen eine zu weit gehende Rekombination des Plasmas festgestellt wird. Auch kann die Elektrodentemperatur einen Einfluss auf die Form des Echosignals 32 haben und somit über die Auswertung des Echosignals ermittelt werden.

Andererseits kann, wenn bei jedem HF-Schwingungspaket eine Neuzündung gewünscht ist, der Abstand zwischen einzelnen Paketen auch soweit vergrößert werden, bis eine ausreichende Rekombination des Plasmas festgestellt wurde.

Weiter ist es möglich, mit der vorbeschriebenen Impulsechomessung subtile dynamische Veränderungen der Verhältnisse an einer Elektrode während eines Behandlungsvorgangs zu erfassen und zur Steuerung der Speiseeinrichtung 21 zu nutzen. Beispielsweise kann bei der Kontaktkoagulation die Elektrode des Instruments zunächst mit feuchtem Gewebe in Kontakt gebracht werden. Das Echosignal 32 hat in diesem Zustand eine charakteristische Form. Sobald durch fortwährende Energiebeaufschlagung der beteiligten Elektrode eine Trocknung der Elektrode und Dampfbildung am Gewebe verzeichnet wird, ändert das Echosignal 32 seine Form auf charakteristische Weise. Die Speiseeinrichtung 21 kann nun ihre Energieabgabe ändern, z.B. die Spannung reduzieren, um zum Beispiel eine nun drohende Funkenbildung zu verhindern. Es können Spitzenspannung und/oder Tastverhältnis oder andere Einflussfaktoren geändert, z.B. reduziert werden. Weil die Form des Echosignals sehr empfindlich auf die Verhältnisse an der Elektrode reagiert, lässt sich jede gewünschte Betriebsart, z.B. die Kontaktkoagulation, mit fortdauerndem Regeleingriff betreiben und dabei der Energieeintrag maximieren, ohne Funkenbildung zu riskieren. Andererseits kann bei Behandlungsmoden, bei denen Funkenbildung gewünscht ist, durch die fortwährende Überprüfung der Form der Echosignale eine gewünschte Betriebsweise, beispielsweise Funkenbildung und Plasmaerhalt bei pulsbreitenmoduliertem HF-Signal unter verschiedenen Prämissen, beispielsweise minimaler Leistung oder maximalem Schneideffekt oder dergleichen, erreicht werden.

Bei dem erfindungsgemäßen Gerät und dem erfindungsgemäßen Verfahren handelt es sich um Behandlung von biologischem Gewebe mit einer Kryospitze; es werden von einem Gerät 16 zu einem Instrument 14 Testsignale 31 gesendet und die entstehenden und danach eintreffenden Echosignale 32 untersucht, um bestimmte Eigenschaften und Veränderung von Eigenschaften an der Leitung 15, dem Instrument 14, dem Gewebe 20 oder auch einem an einer Elektrode des Instruments 14 vorhandenen Fluidkörper zu erfassen und den Betrieb der Speiseeinrichtung 21 entsprechend zu steuern.

### Bezugszeichen:

- 10: Einrichtung
- 11: Patient
- 12: Liege
- 13: Erdpotential
- 14: Instrument / Kryosonde
- 15: Leitung
- 16: Gerät
- 17: Fluidzufuhrleitung
- 18: Fluidrückführungsleitung
- 19: Kryospitze
- 20: Gewebe
- 21: Speiseeinrichtung
- 22: Steuereingang
- 23: Ausgang
- 24: Testsignalsender
- 25: Koppeleinrichtung
- 26: Signaleingang
- 27: Signalausgang
- 28: Echosignalempfänger
- 29: Analyseeinrichtung
- 30: Leiterabschnitt
- 31: Testsignal
- 32: Echosignal
- 33: gefrorenes Gewebe
- 34: Bedienelement
- 35: Induktivität

## Patentansprüche

1. Gerät (16) zur Speisung eines über eine Fluidleitung (15) an das Gerät (16) angeschlossenen medizinischen Instruments (14) mit einem Fluid als Betriebsmedium zur Behandlung von biologischem Gewebe mit einer Kryospitze (19), wobei die Fluidleitung (15) sowohl eine Zufuhrleitung (17) als auch eine Rückführungsleitung (18) umfasst, wobei wenigstens eine der beiden Leitungen (17, 18) elektrisch leitend ausgebildet oder mit einem elektrischen Leiter versehen, so dass die Kryospitze (19), die zum direkten Kontakt mit biologischem Gewebe (20) dient, von einem elektrischen Testsignal (31) erreichbar ist, das von dem Gerät (16) ausgehend über die Leitung (15) zu dem Instrument (14) und dessen Kryospitze (19), und von dieser über die Fluidleitung (15) zurück zu dem Gerät (16) läuft,
mit einem Testsignalsender (24), der dazu eingerichtet ist, die Testsignale (31) an die Fluidleitung (15) abzugeben,
mit einem Echosignalempfänger (28), der dazu eingerichtet ist, ein von einem Testsignal (31) verursachtes Echosignal (32) zu empfangen, und
mit einer Analyseeinrichtung (29), die dazu eingerichtet ist, anhand des Echosignals (32) eine Veränderung eines physikalischen Zustands in oder an dem Instrument (14) und/oder in oder an der Fluidleitung (15) zu erfassen.

2. Gerät nach einem der vorstehenden Ansprüche, **dadurch gekennzeichnet, dass** die Analyseeinrichtung (29) dazu eingerichtet ist, in Reaktion auf eine charakteristische Veränderung des Echosignals (32) eine Aktion auszulösen.

3. Gerät nach Anspruch 2, **dadurch gekennzeichnet, dass** die Aktion ein Aktivieren oder ein Deaktivieren des Instruments (14) umfasst.

4. Gerät nach Anspruch 2 oder 3, **dadurch gekennzeichnet, dass** die Aktion die Erzeugung eines Signals umfasst, das ein angeschlossenes Instrument (14) kennzeichnet.

5. Gerät nach einem der vorstehenden Ansprüche, **dadurch gekennzeichnet, dass** der Testsignalsender (24) dazu eingerichtet ist, gleichspannungsfreie und/oder gleichstromfreie Testsignale zu generieren.

6. Gerät nach einem der vorstehenden Ansprüche, **dadurch gekennzeichnet, dass** der Testsignalsender (24) dazu eingerichtet ist, ein moduliertes HF-Signal abzugeben.

7. Gerät nach einem der vorstehenden Ansprüche, **dadurch gekennzeichnet, dass** der Testsignalsender (24) dazu eingerichtet ist, ein gleichspannungsfreies Impulssignal abzugeben.

8. Verfahren zur Überwachung eines über eine Leitung (15) von dem nach einem der vorstehenden Ansprüche ausgebildeten Gerät (16) gespeisten medizinischen Instruments (14), bei dem:
Elektrische Testsignale (31) an die Fluidleitung (15) gegeben werden, über die das Instrument (14) mit einem Fluid als Betriebsmedium versorgt wird,
wenigstens ein von einem oder mehreren elektrischen Testsignalen (31) verursachtes Echosignal (32) empfangen wird,
das Echosignal (32) einer Analyse unterzogen und aus der Analyse eine Veränderung eines physikalischen Zustands in oder an dem Instrument (14) und/oder in oder an der Fluidleitung (15) erfasst wird.

9. Verfahren nach Anspruch 8, **dadurch gekennzeichnet, dass** als Testsignale (31) gleichspannungsfreie und/oder gleichstromfreie Impulse genutzt werden.

10. Verfahren nach einem der Ansprüche 8 oder 9, **dadurch gekennzeichnet, dass** die Analyse während der Anwendung des Instruments (14) an einem menschlichen oder tierischen Körper durchgeführt wird.

11. Verfahren nach einem der Ansprüche 8 bis 10, **dadurch gekennzeichnet, dass** eine charakteristische Änderung des Echosignals (32) zur Steuerung des Geräts (16) und/oder des Instruments (14) genutzt wird.

## Claims

1. Device (16) for feeding a fluid, as an operating medium for treating biological tissue, to a medical instrument (14) which is connected to the device (16) via a fluid line (15) and has a cryogenic tip (19),
wherein the fluid line (15) comprises both a supply line (17) and a return line (18), wherein at least one of the two lines (17, 18) is configured to be electrically conductive or comprises an electrical conductor so that the cryogenic tip (18), which serves for direct contact with the biological tissue (20), can be reached by an electrical test signal (31) which runs from the device (16) via the line (15) to the instrument (14) and its cryogenic tip (19), and from this back to the device (16) via the fluid line (15), with a test signal emitter (24) which is configured to emit test signals (31) to the fluid line (15),
with an echo signal receiver (28) which is configured to receive an echo signal (32) provoked by the test signal (31), and
with an analysis device (29) which is configured to detect, by means of the echo signal (32), a change of the physical state in or at the instrument (14) and/or in or at the fluid line (15).

2. Device according to one of the preceding claims, **characterised in that** the analysis device (29) is configured to trigger an action in response to a characteristic change in the echo signal (32).

3. Device according to claim 2, **characterised in that** the action comprises activation or deactivation of the instrument (14).

4. Device according to claim 2 or 3, **characterised in that** the action comprises generating a signal which is characteristic of a connected instrument (14).

5. Device according to any of the preceding claims, **characterised in that** the test signal emitter (24) is configured to generate test signals which are free from direct current voltage and/or direct current.

6. Device according to any of the preceding claims, **characterised in that** the test signal emitter (24) is configured to emit a modulated HF signal.

7. Device according to any of the preceding claims, **characterised in that** the test signal emitter (24) is configured to emit a pulse signal which is free from direct current voltage.

8. Method for monitoring a medical instrument (14), which is fed via a line (15) by the device (16) according to any of the preceding claims, in which:
electrical test signals (31) are emitted to the fluid line (15) via which the instrument (14) is fed with a fluid as an operating medium,
at least one echo signal (32) provoked by one or more electrical test signals (31) is received,
the echo signal (32) is subjected to analysis, and from the analysis a change is detected in a physical state in or at the instrument (14) and/or in or at the fluid line (15).

9. Method according to claim 8, **characterised in that** as test signals (31), pulses are used which are free from direct current voltage and/or direct current.

10. Method according to one of claims 8 or 9, **characterised in that** the analysis is carried out during use of the instrument (14) on a human or animal body.

11. Method according to one of claims 8 to 10, **characterised in that** a characteristic change of the echo signal (32) is used to control the device (16) and/or the instrument (14).

## Revendications

1. Appareil (16) pour alimenter un instrument (14) médical raccordé à l'appareil (16) par l'intermédiaire d'un conduit de fluide (15) en un fluide en tant que milieu de fonctionnement pour traiter des tissus biologiques avec une pointe cryogénique (19), dans lequel le conduit de fluide (15) comprend à la fois un conduit d'arrivée (17) et un conduit de retour (18), dans lequel au moins un des deux conduits (17, 18) est réalisé de manière électriquement conductrice ou est pourvu d'un conducteur électrique de sorte que la pointe cryogénique (19), qui sert au contact direct avec des tissus biologiques (20), peut être atteinte par un signal de test (31) électrique, qui se déplace en partant l'appareil (16) en passant par le conduit (15) vers l'instrument (14) et sa pointe cryogénique (19), et depuis celle-ci en passant par le conduit de fluide (15) pour revenir vers l'appareil (16),
avec un émetteur de signaux de test (24), qui est mis au point pour envoyer les signaux de test (31) au conduit de fluide (15),
avec un récepteur de signaux d'écho (28), qui est mis au point pour recevoir un signal d'écho (32) provoqué par un signal de test (31), et
avec un dispositif d'analyse (29), qui est mis au point pour détecter à l'aide du signal d'écho (32) une modification d'un état physique dans ou sur l'instrument (14) et/ou dans ou sur le conduit de fluide (15).

2. Appareil selon l'une quelconque des revendications précédentes, **caractérisé en ce que** le dispositif d'analyse (29) est configuré pour déclencher une action en réponse à une modification caractéristique du signal d'écho (32).

3. Appareil selon la revendication 2, **caractérisé en ce que** l'action comprend une activation ou une désactivation de l'instrument (14).

4. Appareil selon la revendication 2 ou 3, **caractérisé en ce que** l'action comprend la génération d'un signal qui caractérise un instrument (14) raccordé.

5. Appareil selon l'une quelconque des revendications précédentes, **caractérisé en ce que** l'émetteur de signaux de test (24) est mis au point pour générer des signaux de test sans tension continue et/ou sans courant continu.

6. Appareil selon l'une quelconque des revendications précédentes, **caractérisé en ce que** l'émetteur de signaux de test (24) est mis au point pour envoyer un signal HF modulé.

7. Appareil selon l'une quelconque des revendications précédentes, **caractérisé en ce que** l'émetteur de signaux de test (24) est mis au point pour envoyer un signal d'impulsion sans tension continue.

8. Procédé pour surveiller un instrument (14) médical alimenté par l'appareil (16) réalisé selon l'une quelconque des revendications précédentes par l'intermédiaire d'un conduit (15), où :
des signaux de test (31) électriques sont envoyés au conduit de fluide (15), par l'intermédiaire duquel l'instrument (14) est alimenté en un fluide en tant que milieu de fonctionnement,
au moins un signal d'écho (32) provoqué par un ou plusieurs signaux de test (31) électriques est reçu,
le signal d'écho (32) est soumis à une analyse et sur la base de l'analyse, une modification d'un état physique dans ou sur l'instrument (14) et/ou dans ou sur le conduit de fluide (15) est détectée.

9. Procédé selon la revendication 8, **caractérisé en ce que** des impulsions sans tension continue et/ou sans courant continu sont utilisées en tant que signaux de test (31).

10. Procédé selon l'une quelconque des revendications 8 ou 9, **caractérisé en ce que** l'analyse est menée au cours de l'application de l'instrument (14) sur le corps d'un humain ou d'un animal.

11. Procédé selon l'une quelconque des revendications 8 à 10, **caractérisé en ce qu'**un changement caractéristique du signal d'écho (32) est utilisé pour commander l'appareil (16) et/ou l'instrument (14).
